# EUROPEAN PATENT SPECIFICATION

(11) **EP 2 967 758 B1**
(45) Date of publication and mention of the grant of the patent: **07.10.2020**
(21) Application number: 13715619.6
(22) Date of filing: 11.03.2013
(51) Int. Cl.: A61C 5/90, A61C 1/08, A61B 1/24, A61B 1/32

(54) **MODULAR ASSEMBLY FOR DENTAL APPLICATIONS**
MODULARE ANORDNUNG FÜR DENTALE ANWENDUNGEN
ENSEMBLE MODULAIRE POUR APPLICATIONS DENTAIRES

(43) Date of publication of application: 20.01.2016
(73) Proprietor: Planmeca Oy, 00880 Helsinki (FI)
(72) Inventor: LEINO, Juha, FI-01420 Vantaa (FI)
(74) Representative: Heinonen & Co
(86) International application number: PCT/EP2013/054830
(87) International publication number: WO 2014/139552

(56) References cited:
- US-A1- 2010 203 466
- US-A1- 2010 297 579

## Description

### FIELD OF THE INVENTION

Generally invention relates to dental appliances, such as mouth props and cheek retractors and in particular to those, provided with means for mouth cavity illumination.

### BACKGROUND

Preserving patient's mouth stably open during dental procedures and uniform illumination of an oral cavity is essential for successful dental treatment. Lack of appropriate illumination slows down the procedure and increases the risks of inaccuracies, which may lead to unpredictable consequences. While illumination of the anterior teeth during treatment is relatively easy, the problem of providing sufficient light to inner cheek area and, correspondingly, to the buccal surfaces of molar teeth, is not resolvable by traditional means, such as common dentistry lamps.

A number of appliances exist in dental practice for illuminating oral cavity of patient's mouth while keeping lips and cheeks in retracted position. Different implementations and concepts of said appliances vary from handheld illumination devices to mouth props and cheek retractors/expanders equipped with light sources. Also referred as bite blocks, mouth prop devices with integrated illumination means are disclosed in US 2012/0228528 and US 2009/0323370. Cheek retractors provided with in-built light sources are disclosed e.g. in US 2006/0155171 and WO2006/026129. Above mentioned systems, however, are constrained with several common problems. So called bite block devices are traditionally being inserted between molars of upper and lower jaws to prevent the patient to accidentally close his or her mouth during treatment, so illumination provided by said devices is directed mainly to lingual and palatal areas of the teeth, leaving the buccal area of the teeth obscured. Combined cheek retractor-illuminator devices are intended to pull the cheeks aside and hold them during dental treatment, and at the same time to illuminate oral cavity area. All known devices of the kind, however, are provided in one integral piece, which means that they must either be sterilization-resistant or disposable. Continuous daily disposing (including recycling) of dental equipment may not be optimal from the environmental point of view. Moreover, it is a well-known fact that electronic devices are relatively difficult to recycle. From the other hand, exposing devices, containing electronic components, to high temperatures (over 100 °C, routine autoclaving), does not positively affect the lifespan of said devices, which again results in disposing. Either way, the integral retractor-illuminator devices do not provide cost-effective alternative to common illumination means, such as traditional dental operating lamps. In addition, known integral retractor-illuminator devices are implemented on the basis of light sources, usually LEDs, either attached to or encapsulated into plastic casing. Prolonged utilization of such devices (e.g. during dental surgery) may cause unnecessary thermal effects onto any oral tissue and adversely affect the whole procedure.

US 2010/203466 constitutes a prior art of particular relevance and concerns a dental apparatus comprising a frame for preserving a mouth cavity open, two lip and cheek retractors attached to the frame, and light source(s), such as light emitting diode(s) accommodated in each of the lip and cheek retractors. Although indicating that the retractors may be separable from the frame, said publication further teaches that also a power cord, being an integral part of each of the retractors, must be disposed (together with the retractor) after one or several uses. Clearly, such solution will result in higher manufacturing and recycling costs.

Additionally, US 2010/297579 discloses a dental brace adapted for placement between upper and lower jaws during dental procedures, said brace comprising a C-shaped central arm with removable sheath-like members made of rubber and attachable to both ends of the central arm. The brace disclosed hereby provides no hint on solving a problem of illumination during dental procedures.

It is therefore desirable to provide a simple, reliable and cost-effective device and method for illuminating "difficult" areas within the oral cavity of the patient, such as areas between internal surfaces of the cheeks and adjacent teeth, while keeping patient's lips and cheeks retracted. It is further desirable to decrease at least to some extent the number of medical electronic appliances disposed daily.

### SUMMARY OF THE INVENTION

The objective of the present invention is to at least alleviate each of the problems set forth in the background section and to provide a novel modular solution for facilitating dental diagnostics and treatment by supplying means for expanding and retaining an oral cavity exposed during dental procedures while providing sufficient light to otherwise obscured areas within the oral cavity, such as buccal surface of the teeth, for example.

The objective is achieved by different embodiments of an intraoral assembly, said assembly having a modular structure and comprising a frame and a pair of replaceable adapter units, suitable for being detachably mounted onto the frame; each adapter unit is implemented as a discrete piece preferably manufactured from semi-rigid silicone material. Said assembly is further provided with means for enlightening the oral cavity of the patient and in particular buccal surfaces of molar teeth, thus facilitating and possibly accelerating an overall dental procedure.

In one aspect of the invention an assembly for retracing lips and cheeks of a patient and for retaining an oral cavity exposed during dental procedures is provided, according to what is defined in the independent claim 1, said assembly comprises a frame, implemented as a bracket-shaped body supplied with a pair of adapter mount portions obliquely extending sidewards with regards to a horizontal axis of the frame body, and a pair of adapter units, each provided as a discrete piece and adapted to be detachably mounted onto corresponding mount portions of the frame, wherein each adapter unit comprises a blind bore-type recess to incorporate a corresponding mount portion of the frame, and wherein the three-dimensional structure of each adapter unit is adjusted to engage margins of patient's lips at the corners of a mouth.

In preferred embodiment at least one light source is provided within each adapter mount portion of the frame. All electronic components, including light generating and/or emitting means are provided with or within the frame, accordingly.

In some embodiment, the frame is additionally configured to bear connecting means to an external power source, which external power source can be a battery, for example.

In one substantially supplementary embodiment the frame is also supplied with at least one activation/deactivation means for the light source(s) and, possibly, with light intensity regulation controls.

In some embodiment, each adapter unit is manufactured from semi-rigid polymeric material, such as silicone- or modified silicone (e.g. silicone epoxy-) resin. Any other material, suitable for the purposes of the invention may be utilized as well, whether it complies with medical safety requirements. In terms of mechanical properties said material must be capable of preserving its shape while being easily mountable onto the support and removable from the support.

In some other embodiment each adapter unit is manufactured from translucent anti-glare material with milky appearance.

In an alternative embodiment each adapter unit is manufactured from transparent material and is configured to constitute light focusing means, such as lens, for example.

In some embodiment, the frame is configured to be bendable.

In some embodiment, the frame is configured to be length-adjustable.

In another aspect of the invention an adapter unit for the assembly according to the previous aspect is provided, according to what is defined in the independent claim 16.

The utility of the present invention arises from a variety of reasons depending on each particular embodiment thereof. The assembly of the preferred embodiment is implemented on a modular basis; therefore the adapter units are fully detachable from the frame. Since the adapter units do not comprise any electronic components, their further handling, such as sterilization and further recycling, requires significantly less efforts than handling integral devices of the kind. The configuration thus disclosed provides an environmentally friendly solution which omits continuous disposing of electronic appliances. Then again, the manufacturing costs for modular solution proposed are significantly lower comparing to those for related integral devices, since the manufacturing process largely concentrates on the production of the adapter units, which, in turn, can be produced by simple silicone molding technique. Assembling of electronic component's containing frame units can in turn be realized on a separate line by common techniques; which for no special-purpose manufacturing equipment is further required. Silicone adapter units are implemented to withstand multiple sterilizations while preserving original shape thereof. In addition, said adapter units act as heat insulators which prevent possible thermal effects as referred above.

It should be generally understood that adapter units structurally entirely duplicate each other. Their seemingly different graphical appearance results only from spatial arrangement thereof within the assembly. For clarity purposes on those figures, showing more than one adapter unit, some reference numerals are indicated only at one adapter element from the pair, and are not indicated at the other. Those reference numerals, however, equally refer to corresponding structural features of both paired adapter elements, whether illustrated on same figure.

The term "electronic components" refers in present disclosure to any basic electric device, either discrete or as a part of an integrated circuit, including, but not limited to discrete semiconductors, integrated circuits, optoelectronics, capacitors, resistors, sensors, and various connectors and switches; which electric devices may be integrated within the frame element in any configuration, appropriate for technical implementation of the embodiments of present invention.

The term "buccal" refers in this disclosure to the area pertaining to the internal lining of the cheek and the surface of the teeth and/or the gum beside the (internal) lining of the cheek.

The term "paired" is used in this disclosure to indicate a pair (i.e. two) identical items.

The terms "first" and "second" are used herein to distinguish an element from other element and not to denote any particular order or importance if not otherwise explicitly indicated.

Different embodiments of the present invention will become apparent by consideration of the detailed description and accompanying drawings.

### BRIEF DESCRIPTION OF THE DRAWINGS

Fig. 1 illustrates a three-dimensional reconstruction of a modular intraoral assembly in accordance to the embodiments of the invention.
Figs. 2A-D are technical drawings of the modular intraoral assembly of Fig. 1 in various orientations.
Fig. 3 is a partly collapsed view of the modular intraoral assembly.
Fig. 4 is a partly collapsed view of the modular intraoral assembly with illumination means switched on.
Fig. 5 illustrates a fragment of a frame of the modular intraoral assembly and connection thereof to a power source.

### DETAILED DESCRIPTION OF THE EMBODIMENTS

Detailed embodiments of the present invention are disclosed herein with the reference to accompanying drawings. The same reference characters are used throughout the drawings to refer to same members. Following citations are used for the members:
- 10 -: a modular intraoral assembly;
- 11 -: a frame;
- 12 -: an indentation to incorporate a light source;
- 21 -: an adapter unit;
- 21a -: a crescent-shaped portion of the adapter unit;
- 21b -: a projection portion of the adapter unit;
- 22 -: an inflected groove within the crescent-shaped member 21a of the adapter unit;
- 23 -: an aperture of the adapter unit;
- 24 -: a blind bore-type recess within the adapter unit;
- 25 -: apex end of the adapter unit;
- 41 -: a light source/light source panel;
- 51 -: an aperture in the frame;
- 52 -: a loop for guiding power source connecting means;
- 61 -: a power source connecting means;
- 70 -: a power source.

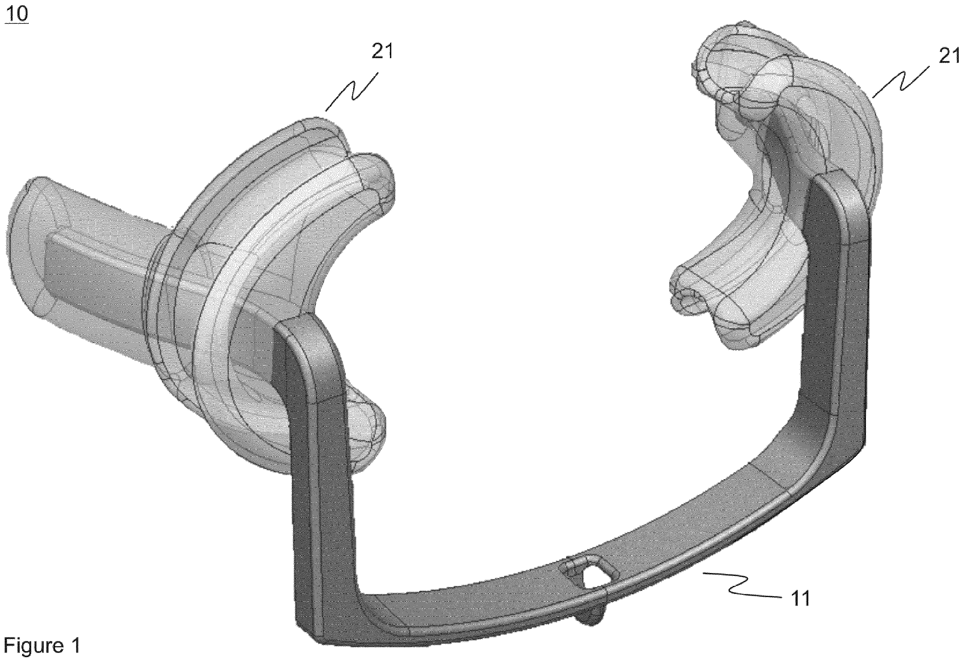

A modular intraoral assembly 10 is provided (Fig.1). The assembly 10 comprises a frame 11 preferably implemented as a multi-junction bracket-like structure, either free end of which structure is arranged to extend obliquely sideways. The assembly 10 further comprises a pair of replaceable adapter units 21, each arranged to be mounted onto a corresponding free end of the frame 11, as shown on Fig.1.

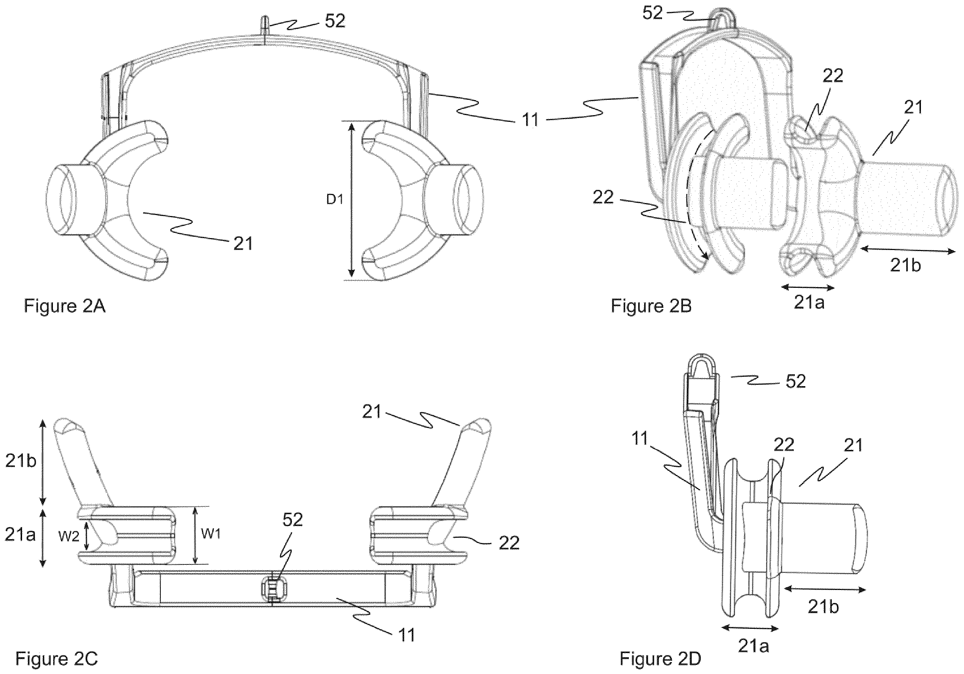

Figures 2A-2D represent the intraoral assembly 10 in various orientations. Fig. 2A thus shows the assembly 10 in "ready-to-use" position with respect to the user. Fig. 2B shows a half-side view of the assembly 10. Fig. 2C shows a top view of the assembly 10; and Fig. 2D shows a side profile of the assembly 10.

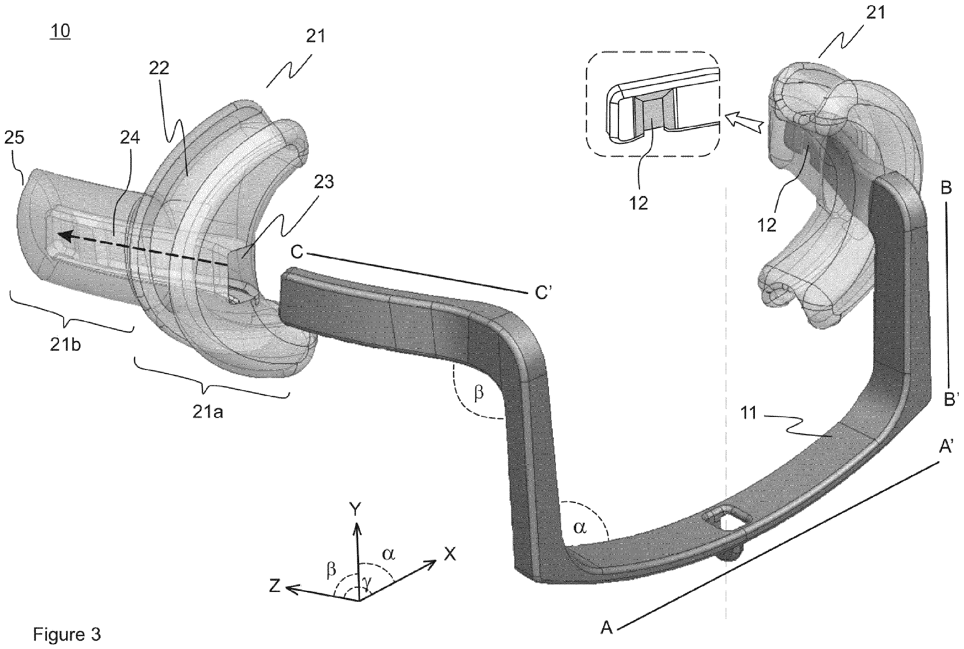

Fig. 3 represents a partly collapsed view of the assembly 10 with one adapter unit 21 detached and the other one mounted onto frame. As already indicated above, the adapter units are provided as discrete pieces which are fully interchangeable. Each adapter unit 21 is configured as a three-dimensional one-piece member, wherein one can distinguish a crescent-shaped portion 21a and a projection portion 21b (see also Figs. 2B-D). Portion 21a may be described as a semi-circular arched object, wherein a groove 22 is arranged over a whole convex surface of the arch (Figs. 2B-D, 3). The same effect is usually achieved when a piece of flexible hose-pipe is cut longitudinally into half and is bent into a crescent so that a groove thus formed defines a convex surface of the crescent. For clarity purposes groove 22 is indicated on Fig. 2B by dashed arrow. Groove 22 therefore has an arched profile and is configured to engage margins of patient's lips at the corners of a mouth. When a pair of adapter units is utilized within the assembly 10, each adapter unit 21 is mounted onto the frame so, that portions 21a and, respectively, grooves 22 of each adapter unit face opposite directions with regards to each other. Such an arrangement enables patient's lip and cheek retraction and further retention of patient's mouth open when the assembly 10 is inserted within a mouth cavity.

Geometrical parameters of the portion 21a, such as a width of the groove and a distance between ends of a bi-concave surface, are indicated by references W1 and W2 on Fig. 2C and D1 on Fig. 2A. W1 and W2 (Fig. 2C) thus refer to distances between outer- and inner surfaces defining walls of the groove 22, and those are equal approximately 15-20 mm and 10-15 mm, respectively. Distance between crescent corns D1 (Fig. 2A) equals approximately 45-55 mm, preferably 49-51 mm. Those values are, however, exemplary and refer to one technical implementation in accordance to some embodiment. Mentioned values may vary dependent on design of the final product.

Each adapter unit 21 is further provided with a substantially ellipse-shaped projection 21b, configured as a hollow body appended to a concave surface of the arched portion 21a (Fig. 3), as described further. Projection 21b is configured to extend into patient's mouth cavity and pertain to an inside of the cheek.

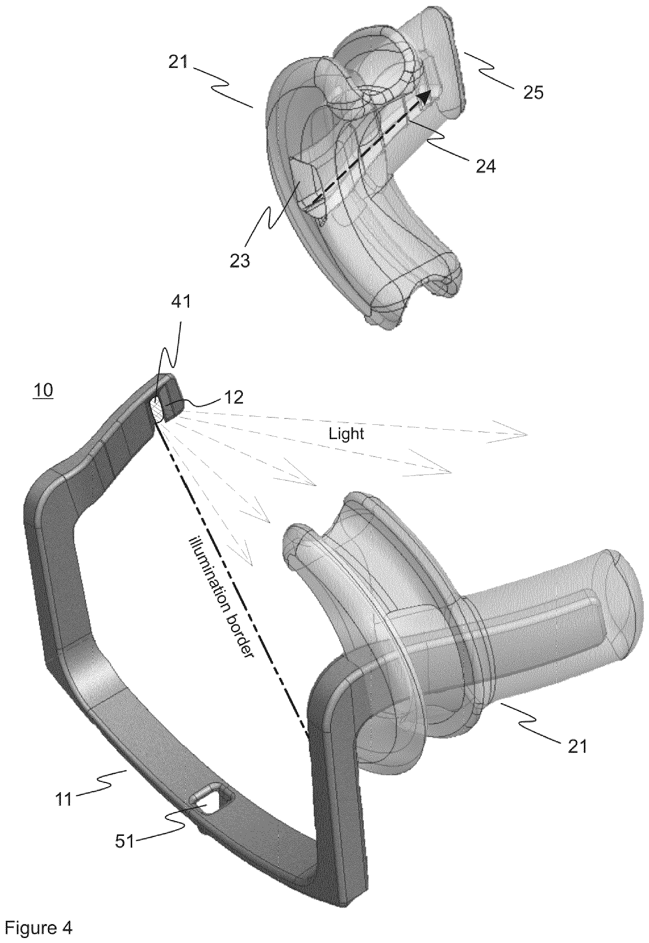

Projection part 21b of each adapter unit 21 comprises an apex end 25 and a base, opposite to the apex end, which base comprises an aperture 23. Aperture 23 in turn provides an entrance into a blind bore-type (i.e. closed from one end) recess 24 arranged within the projection part 21b of the adapter unit 21 (recess 24 is shown by dashed arrow on Figs. 3 and 4). Recess 24 does not form through hole. Blind end of said recess may thus be located either substantially in the middle of the projection 21b or more proximally to an apex end 25 thereof. Base of the projection part 21b therefore locates in a concave of the crescent-shaped part 21b and borders upon arc edge proximal to the frame.

It is to be understood, that adapter units 21 are provided as integral members, each manufactured as an entity by means of i.e. silicone molding, therefore differentiation of parts 21a and 21b is merely functional and serves clarity purposes.

Fig. 3 schematically illustrates how the adapter unit 21 is mounted onto the frame 11. Frame 11 is provided herein as a multi-junction bracket-like structure, wherein the term "multi-junction" is utilized to indicate more than two junction points between structural elements located in different spatial planes. Junction points are indicated on Fig. 3 by reference angles alpha and beta. Frame structure is preferably manufactured as a solid integral piece with no pivot connections.

The multi-junction bracket-like frame 11 comprises bridge A-A', with two arms, each indicated as B-B' and arranged at a slightly obtuse alpha angle with regards to bridge A-A'. An alpha angle formed between A-A' and B-B' is generally adjusted to be equal or greater than 90°, but smaller than 145°, preferably greater than 90° but smaller than 120°. Frame 11 further comprises two arms C-C', each arranged at an obtuse beta angle with regards to corresponding arms B-B', which beta angle is adjusted between 90 and 160°. Each arm C-C' thus forms a support structure for mounting an adapter unit 21 thereto, as illustrated by Fig. 3. Provision of the adapter units 21 is such, that tight fixation thereof on the frame arms C-C' is provided, as disclosed further.

Arms C-C' of the frame 11, thus adapted to receive adapter units 21, may further be referred as "adapter mount portions". Spatial arrangement of adapter mount portions with regards to bridge A-A' of the frame may be explained in terms of geometry in the following way. If one assume, that bridge A-A' and arms B-B' and C-C' are positioned along axes x, y and z, respectively, wherein axes x and z share the same plane, a gamma (γ) -angle, formed between x and z, will reflect an angular position of arm C-C' with regards to bridge A-A'. Gamma angle is thus adjusted between 90 and 145 °, preferably greater than 90° but smaller than 120°. Once an angle between axes z and x on plane is determined, arm C-C' is simply translated vertically upwards along axis y on a distance defined by the length of arm B-B'. Bridge A-A' and each arm C-C' lie therefore in two substantially parallel horizontal planes, wherein distance between planes is defined by the length of arm B-B'. Mirror symmetry exists between two halves of the frame 11 (center of symmetry is indicated on Fig. 3 by vertical dashed line).

As illustrated by Fig. 3, each adapter mount portion C-C' of the frame 11 is adapted to receive an adapter unit 21 so, that when two adapter units are mounted onto the frame, grooves 22 face opposite directions with regards to each other. Such adapter-to-frame configuration naturally provides means for spreading corners of patient's lips sideways so that lip margins on the left and on the right cheek sides are being engaged by grooves 22 of crescent-shaped adapter portions 21a.

Manufacturing of the adapter units 21 from semi-rigid silicone material, in accordance to one embodiment, enables a so called "pull-on/pull-off' mounting/dismounting technique, wherein each adapter unit 21 may be simply pulled onto a corresponding mount portion of the frame 11 through the aperture 23 thereof. Elongated arm C-C' is thus adapted to receive an ellipse-shaped projection portion 21b of the adapter unit. Semi-rigid nature of silicone material, the adapter units are manufactured from, and technical features of said adapter units (i.e. inner diameter of the recess 24) ensure tight fitting of each adapter unit onto the frame.

Procedure of dismounting of the adapter units from the frame 11 is implemented so, that each adapter unit 21 is pulled off the frame by hand, like a sheath is being pulled off a knife, for example. Care must be taken when pulling adapter units off the frame, in order to preserve an internal structure of the adapter unit undamaged; adapter units thus must not be torn off the frame with force. Utilized adapter units may be sterilized by autoclaving. The frame may be cleaned by a sterilizing agent, for example.

A small ledge (not shown) may be arranged on each the adapter mount portion of the frame in order to provide a "stop-mark" -type indication that each adapter unit is correctly mounted onto the frame.

Until now a provision of a modular assembly for patient's lip- and cheek retracting and expanding of mouth cavity during dental procedures has been disclosed. In accordance to the preferred embodiment, the assembly 10 is provided with means for illuminating an oral cavity of the patient, and especially, for illuminating otherwise obscured areas, such as a cavity between an internal cheek lining and buccal surfaces of molar teeth.

To cope with the problem set above (illumination of obscured areas within mouth cavity), each adapter mount portion (defined by arm C-C', Fig. 3) of the frame 11 is provided with at least one light source 41 (Fig. 4). Light source 41 may be completely or partially enclosed within each adapter mount portion of the frame 11. Whether more than one light source is utilized (e.g. an array of light emitting diodes), said light sources may be encapsulated within a tubular or flat casing or panel, to which reference numeral 41 will refer as well.

Light source 41 is preferably integrated directly to the adapter mount portion of the frame next to the distal end thereof. In order to receive the light source, an indentation 12 of the appropriate size is preferably provided at the interior surface of each adapter mount portion of the frame (Fig. 3, dashed box). Indentation 12 is arranged such, that three slanted surfaces would surround the light source 41, when the latter is fixed into place. The purpose of indentation 12 is to prevent formation of shadows.

Light source 41 is preferably realized on the basis of light emitting diodes (LEDs), however any other appropriate light emitting technology may be utilized. For example, light-fiber based light guide technology may be utilized as well; however, presence of light source within the frame will then be required.

Light source 41 is preferably adapted to emit white light. However, a visible radiation (light) source emitting at any other wavelength may be utilized whether appropriate for the purposes of the invention. Illumination power can in general be adjusted suitable for any dental procedure, including among others dental diagnostics, such as for caries or dental calculus.

In some embodiment the adapter units 21 are manufactured from substantially translucent anti-glare silicone material with so called "milky" appearance. The term "translucent" is utilized herein to designate a material with substantially semi-transparent appearance, which is still able to partially transmit or diffuse light. Whether a pair of above said anti-glare adapter units is mounted onto frame and the corresponding light sources are switched on, light emitted by said light sources encounters translucent material and undergoes subsurface scattering, deflecting in any direction. As a result, a mild but intensive enlightening of the oral cavity is provided, which omits dazzling effects and is not disturbing for clinician's eyes. An essential advantage of utilizing "milky" translucent material is such that using un-shaded light sources during dental procedures may not only be disturbing, but also harmful for eyes of dentist and dental assistant, i.e. light sources currently utilized in dentistry are of such a power, that may injure eyes of medical personnel when utilized on a constant basis. Provision of the adapter unit in accordance to present embodiment resolves this problem.

In an alternative embodiment the adapter units 21 are manufactured from an essentially transparent, light transmissive material such, that provision of each adapter unit constitutes light focusing means, in the form of lens, for example. Lens may be arranged such, to confine light emitted by the light source 41 strictly to inside of the mouth cavity, as schematically indicated on Fig. 4 (dashed line marked as "illumination border"), at the same time providing uniform distribution of emitted light rays therewithin. It is important to understand, however, that illumination border thus indicated on Fig. 4 can only be created by means of lens technology provided by the adapter unit 21, whereas Fig. 4 for clarity purposes shows illumination border in an absence of said adapter unit (adapter unit on the left is detached). In practice illumination border can appear only when the adapter unit, provided in accordance with the present embodiment, is mounted onto the corresponding frame portion. Provision of the adapter unit in accordance to this embodiment enables attaining required level of luminous efficiency with less power consumption.

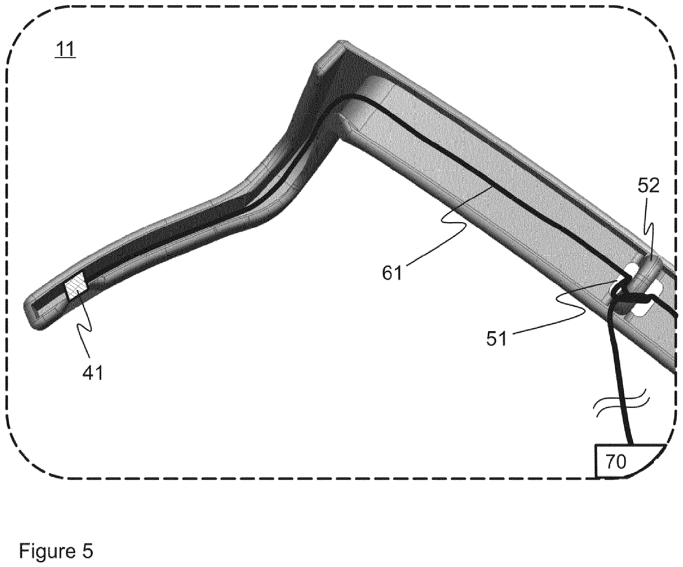

In some embodiment, the frame 11 is configured to bear connecting means 61 to an external power source 70, such as an external battery, for example (Fig. 5). Technical implementation of the assembly 10 preferably omits utilization of a built-in power source, which otherwise would increase construction's weight and therefore result in certain discomfort for patients. The other reason to utilize an external power source is its capacity. Since powering of white light emitting diodes, utilized as or within light sources in accordance to some embodiment, requires a certain extent of energy, the choice of a power source must also be appropriate. Said external power source may be of any known battery type, functionally suitable for the purposes of the invention. The above said connecting means for an external power source may thus be realized as a power cord, cable or wire, and it may be integrated within or attached onto the frame. Fig. 5 thus shows a fragment of the frame 11, provided as a partially open structure, wherein a wire 61 extends from the light source 41 to the external power source 70. Wire 61 may be covered by a protective cover or, alternatively, coated by polymeric layer (e.g. polyurethane).

Whether the connecting means 61 are attached to or integrated within the frame 11, the frame 11 is configured to comprise an aperture 51 (Figs. 4, 5) to let the connecting means 61 through. In accordance to some embodiment frame 11 also comprises a loop 52 for guiding wires 62 towards power source 71. The frame 11 may additionally be provided with at least one fixation element, such as a clip, a loop and the like in order to keep the connecting means properly arranged. In some supplementary embodiment an additional clip or clips may be attached to above said connection means for clipping it to patient's garment.

In some embodiment, the frame 11, and especially the bridge A-A' of the frame 11, is configured to be bendable. Frame 11 may thus be manufactured from a substantially resilient material, which allows, at least to some extent, bending of the frame along a horizontal bridge A-A' thereof. However, since the frame 11 is intended to serve as means for holding patient's lips and cheeks in retracted position, resiliency thereof is to be restricted. The horizontal bridge A-A' of the frame 11 is thus bendable to such an extent, that the assembly 10 may be positioned (partially) within patient's mouth, so that the margins of patient's lips at the corners of a mouth become engaged by the grooves 22 of each adapter unit 21 of the assembly 10. Deflection angle of the bridge A-A' during bending may therefore not exceed 45 degrees, preferably ranging between 5 and 45 degrees. For this reason, the frame 11 in accordance to this embodiment is preferably manufactured in at least two sizes, for adults and for children, respectively. The term "deflection angle" is utilized herein to indicate a bending angle for an elongated flat object, said angle being formed substantially in the center of the elongated flat object when force is applied onto the edges thereof.

Bridge A-A' is referred in this disclosure as an essentially flat horizontal object, however, technical implementation of the frame 11 enables provision of a slightly bi-concave or substantially s-shaped bridge A-A'.

In an alternative embodiment, the frame 11 of the assembly 10 is configured to be length-adjustable. Technical implementation of the frame in accordance with this embodiment includes equipping the frame by means, enabling altering the length of the bridge A-A' by several centimeters. This procedure allows manual adjusting the distance between adapter mount portions, thus enabling implementation of frame 11 in one size. Length adjustment means may be realized on the same basis, as for common studio head-phone's size adjustment means, for example. In practice, however, an embodiment disclosing utilization of bendable frame manufactured in smaller and bigger sizes proved to be more reliable.

In one substantially supplementary embodiment the frame 11 may be provided with at least one activation/deactivation means for the light sources 41, realized preferably as an ON/OFF switch. Said means may further be equipped with light intensity regulation controls. Alternatively light sources may be activated by connecting the powering wires, as disclosed above, to an external power source; or the activation may be achieved by first connecting the wires and then switching the power source on. For the purposes of the invention simultaneous activation of both light sources is required, therefore above mentioned switch is preferably adapted to activate/deactivate light sources 41 on both frame sides at once.

The modular intraoral assembly 10 may be utilized in dental practice in accordance with the following steps:
a) obtaining a frame 11 and a pair of sterilized adapter units 21;
b) mounting the first adapter unit and the second adapter unit onto corresponding adapter mount portions (arms C-C') of the frame 11 by means of "pull-on" technique so, that when both adapter units are mounted onto the frame, grooves 22 thereof face opposite directions;
c) placing thus obtained assembly 10 into patient's mouth such, that the margins of patient's lips on the right and left cheek sides become engaged by grooves 22 of the adapter units 21, while projections 21b pertain to the inside of the cheeks; and that the bridge A-A' as well as arms B-B' of the frame 11 remain outside the patient's mouth and thus serve as means for holding patient's lips and cheeks in retracted position;
d) activating the light sources 41;
e) deactivating the light sources 41;
f) withdrawing the assembly 10 from patient's mouth;
g) dismounting adapter units 21 from the frame by pulling them off the frame.

Necessary dental operations ought to be performed between steps d and e; however, since the assembly of the present invention does not directly relate to executing dental procedures, the step indicative thereof has been omitted from the list above.

## Claims

1. A modular assembly (10) for dental applications comprising cheek and lip retracting means and a frame for preserving a mouth cavity open, **characterized in that**
- cheek and lip retracting means are implemented in the form of a pair of detachable and replaceable adapter units (21);
- the frame (11) is a multi-junction bracket-shaped member with a bridge A-A' lying in a first horizontal plane and two adapter mount portions C-C' each positioned obliquely sideways with regards to bridge A-A' and lying in a second horizontal plane;
- adapter mount portions C-C' are connected to opposite ends of the bridge A-A' by arms B-B';
- each adapter mount portion is configured to accommodate at least one light source (41);
- each adapter unit (21) comprises a semi-circular arched member (21a) with a groove (22) arranged over a whole convex surface of the arch, and a projection (21b) appended to a concave surface of the arch;
- each adapter unit (21) comprises an aperture (23) and a blind bore-type recess (24), via which each adapter unit (21) is mounted onto a corresponding adapter mount portion of the frame;
- each adapter mount portion is configured to receive a corresponding adapter unit (21) so, that corresponding light sources (41) are encased by projections (21b) of each adapter unit and grooves (22) of each adapter unit face opposite directions.

2. The assembly of claim 1, wherein two halves of the frame (11) are interrelated in terms of mirror symmetry.

3. The assembly of claim 1, wherein a pair of adapter units (21) is interrelated in terms of mirror symmetry, and wherein adapter units (21) within said pair are symmetrical duplicates of each other.

4. The assembly of any preceding claim, wherein each adapter unit (21) is manufactured from semi-rigid polymeric material.

5. The assembly of claim 4, wherein the semi-rigid material is silicone resin.

6. The assembly of claim 4, wherein the semi-rigid material is modified silicone resin.

7. The assembly of any of the claims 5 or 6, wherein the adapter unit (21) is manufactured from semi-rigid translucent antiglare silicone material with milky appearance.

8. The assembly of any of the claims 5 or 6, wherein each adapter unit (21) is manufactured from essentially transparent, light transmissive material and wherein provision of each adapter unit constitutes a lens.

9. The assembly of any preceding claim, wherein each light source (41) is located within the corresponding indentation (12) and is arranged to emit light into a mouth cavity.

10. The assembly of any preceding claim, further provided with means (61) connecting light source (41) to an external power source (70), said connecting means (61) implemented preferably in the form of a power cord, a cable or a wire.

11. The assembly of claim 10, wherein an external power source is a battery.

12. The assembly of any preceding claim, wherein the light source (41) comprises at least one light emitting diode.

13. The assembly claim 12, wherein the light emitting diode is a white light emitting diode.

14. The assembly of any preceding claim, wherein the frame (11) is implemented as a bendable element, which flexibility is defined by a deflection angle of the bridge A-A' and ranges between 5 and 45 degrees.

15. The assembly of claims 1-13, wherein the frame (11) is provided with length-adjusting means.

16. An adapter unit (21) for assembly (10), comprising a semi-circular arched member (21a) with a groove (22) arranged over a whole convex surface of the arch, **characterized in that** said adapter unit (21) further comprises a projection (21b) appended to a concave surface of the arch and provided with an aperture (23) and a blind bore-type recess (24).

## Patentansprüche

1. Modulare Baugruppe (10) für zahnmedizinische Anwendungen, die Wangen- und Lippenrückzugsmittel und einen Rahmen zum Offenhalten einer Mundhöhle umfasst, **dadurch gekennzeichnet, dass**
- die Wangen- und Lippenrückzugsmittel in der Form eines Paares von lösbaren und ersetzbaren Adaptereinheiten (21) umgesetzt sind,
- der Rahmen (11) ein bügelförmiges Mehrverbindungselement mit einer Brücke A-A', die in einer ersten horizontalen Ebene liegt, und zwei Adapteranbringungsabschnitten C-C', die jeweils schräg seitwärts in Bezug auf die Brücke A-A' angeordnet sind und in einer zweiten horizontalen Ebene liegen, ist,
- die Adapteranbringungsabschnitte C-C' durch Arme B-B' mit entgegengesetzten Enden der Brücke A-A' verbunden sind,
- jeder Adapteranbringungsabschnitt dafür konfiguriert ist, wenigstens eine Lichtquelle (41) aufzunehmen,
- jede Adaptereinheit (21) ein halbkreisförmiges gewölbtes Element (21a) mit einer Rille (22), die über eine gesamte konvexe Fläche des Bogens angeordnet ist, und einen Vorsprung (21b), der an eine konkave Fläche des Bogens angehängt ist, umfasst,
- jede Adaptereinheit (21) eine Öffnung (23) und eine Aussparung (24) in der Art einer blinden Bohrung umfasst, über die jede Adaptereinheit (21) an einem entsprechenden Adapteranbringungsabschnitt des Rahmens angebracht ist,
- jeder Adapteranbringungsabschnitt dafür konfiguriert ist, eine entsprechende Adaptereinheit (21) aufzunehmen, so dass Lichtquellen (41) durch Vorsprünge (21b) jeder Adaptereinheit umhüllt werden und Rillen (22) jeder Adaptereinheit in entgegengesetzte Richtungen zeigen.

2. Baugruppe nach Anspruch 1, wobei zwei Hälften des Rahmens (11) in Bezug auf Spiegelsymmetrie miteinander in Wechselbeziehung stehen.

3. Baugruppe nach Anspruch 1, wobei ein Paar von Adaptereinheiten (21) in Bezug auf Spiegelsymmetrie miteinander in Wechselbeziehung steht und wobei die Adaptereinheiten (21) innerhalb des Paares symmetrische Duplikate voneinander sind.

4. Baugruppe nach einem der vorhergehenden Ansprüche, wobei jede Adaptereinheit (21) aus halbstarrem Polymermaterial gefertigt ist.

5. Baugruppe nach Anspruch 4, wobei das halbstarre Material Silikonharz ist.

6. Baugruppe nach Anspruch 4, wobei das halbstarre Material modifiziertes Silikonharz ist.

7. Baugruppe nach einem der Ansprüche 5 oder 6, wobei die Adaptereinheit (21) aus halbstarrem durchscheinendem blendfreiem Silikonmaterial mit milchigem Erscheinungsbild gefertigt ist.

8. Baugruppe nach einem der Ansprüche 5 oder 6, wobei jede Adaptereinheit (21) aus im Wesentlichen durchsichtigem, lichtdurchlässigem Material gefertigt ist und wobei die Bereitstellung jeder Adaptereinheit eine Linse erzeugt.

9. Baugruppe nach einem der vorhergehenden Ansprüche, wobei jede Lichtquelle (41) innerhalb der entsprechenden Vertiefung (12) positioniert ist und dafür angeordnet ist, Licht in die Mundhöhle abzustrahlen.

10. Baugruppe nach einem der vorhergehenden Ansprüche, die ferner mit Mitteln (61) versehen ist, welche die Lichtquelle (41) mit einer äußeren Energiequelle (70) verbinden, wobei die Verbindungsmittel (61) vorzugsweise in der Form einer Anschlussschnur, eines Kabels oder eines Drahtes umgesetzt ist.

11. Baugruppe nach Anspruch 10, wobei die äußere Energiequelle eine Batterie ist.

12. Baugruppe nach einem der vorhergehenden Ansprüche, wobei die Lichtquelle (41) eine Licht emittierende Diode ist.

13. Baugruppe nach Anspruch 12, wobei Licht emittierende Diode eine weißes Licht emittierende Diode ist.

14. Baugruppe nach einem der vorhergehenden Ansprüche, wobei der Rahmen (11) als ein biegsames Element umgesetzt ist, dessen Flexibilität durch einen Biegewinkel der Brücke A-A' definiert wird und zwischen 5 und 45 Grad beträgt.

15. Baugruppe nach einem der Ansprüche 1 bis 13, wobei der Rahmen (11) mit Längeneinstellungsmitteln versehen ist.

16. Adaptereinheit (21) für eine Baugruppe (10), wobei die Adaptereinheit ein halbkreisförmiges gewölbtes Element (21a) mit einer Rille (22), die über eine gesamte konvexe Fläche des Bogens angeordnet ist, umfasst, **dadurch gekennzeichnet, dass** die Adaptereinheit (21) ferner einen Vorsprung (21b), der an eine konkave Fläche des Bogens angehängt ist und mit einer Öffnung (23) und einer Aussparung (24) in der Art einer blinden Bohrung versehen ist, umfasst.

## Revendications

1. Ensemble modulaire (10) pour applications dentaires, comprenant des moyens de rétraction des joues et des lèvres et un cadre pour garder une cavité buccale ouverte, **caractérisé en ce que**
- les moyens de rétraction des joues et des lèvres sont mis en œuvre sous la forme d'une paire d'unités d'adaptation détachables et remplaçables (21) ;
- le cadre (11) est un élément en forme de support à jonctions multiples doté d'un pont A-A' se situant dans un premier plan horizontal et de deux sections de montage d'adaptateur C-C' positionnées chacune à l'oblique latéralement par rapport au pont A-A' et se situant dans un second plan horizontal ;
- les sections de montage d'adaptateur C-C' sont connectées à des extrémités opposées du pont A-A' par des bras B-B' ;
- chaque section de montage d'adaptateur est conçue pour loger au moins une source lumineuse (41) ;
- chaque unité d'adaptateur (21) comprend un élément arqué semi-circulaire (21a) comportant une gorge (22) disposée sur la totalité d'une surface convexe de l'arc, et une saillie (21b) rattachée à une surface concave de l'arc ;
- chaque unité d'adaptateur (21) comprend une ouverture (23) et un retrait de type trou borgne (24) par l'intermédiaire duquel ladite unité d'adaptateur (21) est montée sur une section correspondante de montage d'adaptateur du cadre ;
- chaque section de montage d'adaptateur est conçue pour recevoir une unité d'adaptateur correspondante (21) de sorte que les sources de lumière correspondantes (41) sont renfermées par des saillies (21b) de chaque unité d'adaptateur et que les gorges (22) de chaque unité d'adaptateur sont tournées dans des directions opposées.

2. Ensemble selon la revendication 1, dans lequel deux moitiés du cadre (11) sont en interrelation en termes de symétrie miroir.

3. Ensemble selon la revendication 1, dans lequel une paire d'unités d'adaptateur (21) est en interrelation en termes de symétrie miroir, et les unités d'adaptateur (21) de ladite paire sont des copies symétriques l'une de l'autre.

4. Ensemble selon l'une quelconque des revendications précédentes, dans lequel chaque unité d'adaptateur (21) est fabriquée à partir de matériau polymérique semi-rigide.

5. Ensemble selon la revendication 4, dans lequel le matériau semi-rigide est de la résine de silicone.

6. Ensemble selon la revendication 4, dans lequel le matériau semi-rigide est de la résine de silicone modifiée.

7. Ensemble selon l'une quelconque des revendications 5 ou 6, dans lequel l'unité d'adaptateur (21) est fabriquée à partir de matériau à base de silicone anti-éblouissement translucide semi-rigide ayant une apparence laiteuse.

8. Ensemble selon l'une quelconque des revendications 5 ou 6, dans lequel chaque unité d'adaptateur (21) est fabriquée à partir de matériau transmetteur de lumière substantiellement transparent et la prévision de chaque unité d'adaptateur constitue une lentille.

9. Ensemble selon l'une quelconque des revendications précédentes, dans lequel chaque source lumineuse (41) se trouve dans l'indentation correspondante (12) et est conçue pour émettre de la lumière dans une cavité buccale.

10. Ensemble selon l'une quelconque des revendications précédentes, pourvue en outre d'un moyen (61) de connexion de la source lumineuse (41) à une source de courant externe (70), ledit moyen de connexion (61) étant mis en œuvre de préférence sous la forme d'un cordon électrique, d'un câble ou d'un fil.

11. Ensemble selon la revendication 10, dans lequel une source de courant externe est une batterie.

12. Ensemble selon l'une quelconque des revendications précédentes, dans lequel la source lumineuse (41) comprend au moins une diode électroluminescente.

13. Ensemble selon la revendication 12, dans lequel la diode électroluminescente est une diode émettrice de lumière blanche.

14. Ensemble selon l'une quelconque des revendications précédentes, dans lequel le cadre (11) et mis en œuvre sous forme d'un élément pliable dont la flexibilité est définie par un angle de déflexion du pont A-A' et va de 5 à 45 degrés.

15. Ensemble selon les revendications 1 à 13, dans lequel le cadre (11) est pourvu d'un moyen de réglage de longueur.

16. Unité d'adaptateur (21) pour ensemble (10), comprenant un élément arqué semi-circulaire (21a) doté d'une gorge (22) disposée sur la totalité d'une surface convexe de l'arc, **caractérisé en ce que** ladite unité d'adaptateur (21) comprend en outre une saillie (21b) rattachée à une surface concave de l'arc et pourvue d'une ouverture (23) et d'un retrait de type trou borgne (24).
